(19) Europäisches Patentamt
European Patent Office
Office européen des brevets

(11) Veröffentlichungsnummer: **0 001 248**
**B1**

(12) **EUROPÄISCHE PATENTSCHRIFT**

(21) Anmeldenummer: **78100856.0**

(22) Anmeldetag: **09.09.78**

(51) Int. Cl.³: **C 07 D 5 13/04,**
B 41 M 5/12,
B 41 M 5/26, //
C 07 D 277/42,
(C 07 D 513/04, 311/00,
277/00)

(54) **Benzopyranothiazole, ihre Herstellung und ihre Verwendung in druck- oder wärmeempfindlichem Aufzeichnungsmaterial**

(30) Priorität: **19.09.77 CH 11405/77**

(43) Veröffentlichungstag der Anmeldung:
**04.04.79 Patentblatt 79/07**

(45) Bekanntmachung des Hinweises auf die Patenterteilung:
**01.10.80 Patentblatt 80/20**

(84) Benannte Vertragsstaaten:
**BE CH DE FR GB**

(56) Entgegenhaltungen:
**FR - A - 2 353 554**
**US - A - 312 109**

(73) Patentinhaber: **CIBA-GEIGY AG**
**Patentabteilung Postfach**
**CH - 4002 Basel (CH)**

(72) Erfinder: **Petitpierre, Jean Claude, Dr.**
**Aeussere Reben 322**
**CH - 4303 Kaiseraugst (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 001 248 B1

Courier Press, Leamington Spa, England.

Benzopyranothiazole, ihre Herstellung und ihre Verwendung in druck- oder wärmeempfindlichem Aufzeichnungsmaterial

Die vorliegende Erfindung betrifft Benzopyranothiazole, Verfahren zu ihrer Herstellung und ihre Verwendung als Farbbildner in druckempfindlichen oder wärmeempfindlichen Aufzeichnungsmaterialien.

Die neuen Benzopyranothiazole entsprechen der Formel

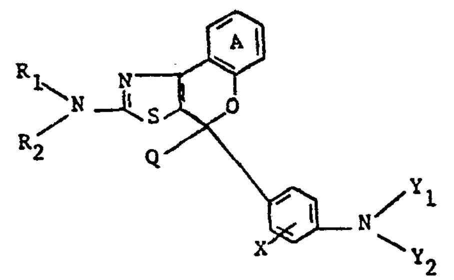

(1)

worin $R_1$ und $R_2$ je Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl Benzyl oder durch Halogen, Niederalkyl oder Niederalkoxy substituiertes Phenyl oder Benzyl oder

$R_1$ und $R_2$ zusammen mit dem sie verbindenden Stickstoffatom einen 5- oder 6-gliedrigen, heterocyclischen Rest oder einen N-Carbazolylrest, Q Wasserstoff, Niederalkyl, Benzyl oder eine Gruppe der Formeln (1a) oder (1b)

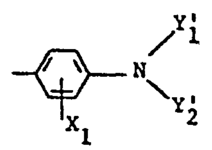

oder

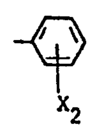

(1a) (1b)

X, $X_1$ und $Z_2$ je Wasserstoff, Halogen, Niederalkyl oder Niederalkoxy, $Y_1$, $Y_2$, $Y_1'$ und $Y_2'$, je Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl, Benzyl oder durch Halogen, Niederalkyl oder Niederalkoxy substituiertes Phenyl oder Benzyl oder die Substituentenpaare $Y_1$ und $Y_2$ und $Y_1'$ und $Y_2'$ unabhängig voneinander je zusammen mit dem sie verbindenden Stickstoffatom einem 5- oder 6-gliedrigen, heterocyclischen Rest oder einen N-Carbazolylrest bedeuten und der Ring A durch Halogen, Nitro, Niederalkyl, Niederalkoxy, Phenoxy oder eine gegebenenfalls durch Niederalkyl, Phenyl oder Benzyl substituierte Aminogruppe weiter substituiert sein kann.

Niederalkyl oder Niederalkoxy stellen bei der Definition der Reste der Benzopyranothiazole in der Regel solche Gruppen dar, die 1 bis 5, insbesondere 1 bis 3 Kohlenstoffatome aufweisen, wie z.B. Methyl, Aethyl, n-Propyl, Isopropyl, n-Butyl, sek.-Butyl oder Amyl bzw. Methoxy, Aethoxy oder Isopropoxy.

Stellen die Substituenten $R_1$, $R_2$, $Y_1$, $Y_2$, $Y_1'$ und $Y_2'$ Alkylgruppen dar, so können sie geradkettig oder verzweigt sein. Beispiele für solche Alkylreste sind Methyl, Aethyl, n-Propyl, Isopropyl, n-Butyl, sek-Butyl, n-Hexyl, n-Octyl oder n-Dodecyl.

Sind die Alkylreste in $R_1$, $R_2$, $Y_1$, $Y_2$, $Y_1'$ und $Y_2'$ substituiert, so handelt es sich vor allem um Cyanoalkyl, Halogenalkyl, Hydroxyalkyl, Alkoxyalkyl jeweils mit insgesamt vorzugsweise 2 bis 4 Kohlenstoffatomen, wie z.B. $\beta$-Cyanoäthyl, $\beta$-Chloräthyl, $\beta$-Hydroxyäthyl, $\beta$-Methoxyäthyl oder $\beta$-Aethoxyäthyl.

Beispiele für Cycloalkyl in der Bedeutung der R-und Y-Reste sind Cyclopentyl oder vorzugsweise Cyclohexyl.

Bevorzugte Substituenten in der Benzyl- und Phenylgruppe der R- und Y-Reste sind z.B. Halogene, Methyl oder Methoxy. Beispiele für derartige araliphatische bzw. aromatische Reste sind p-Methylbenzyl, o- oder p-Chlorbenzyl, o- oder p-Tolyl, Xylyl, o-, m- oder o-Chlorbenzyl oder o- oder p-Methoxyphenyl.

Wenn die Substituentenpaare $R_1$ und $R_2$, $Y_1$ und $Y_2$ und $Y_1'$ und $Y_2'$ zusammen mit dem geinsamen Stickstoffatom einen heterocyclischen Rest darstellen, so ist dieser beispielsweise Pyrrolidino, Piperidino, Pipecolino, Morpholino, Thiomorpholino oder Piperazino.

Die Substituenten $R_1$ und $R_2$ sind vorzugsweise Benzyl oder Niederalkyl.

Der Rest Q bedeutet vorteilhafterweise die Gruppe der Formel (1a). X, $X_1$ und $X_2$ sind vorzugsweise Wasserstoff oder auch Halogen, Methyl, Methoxy oder Aethoxy.

Der Ring A ist vorzugsweise nicht weiter substituiert oder durch Halogen, Niederalkyl oder Niederalkoxy, z.B. durch Chlor, Methyl, tert-Butyl oder Methoxy weiter substituiert.

Halogen in Verbindung mit den vorstehenden Substituenten der Formel (1) ist beispielsweise

Fluor, Brom oder vorzugsweise Chlor.

Praktisch wichtige Farbbildner der Formel (1) entsprechen der Formel

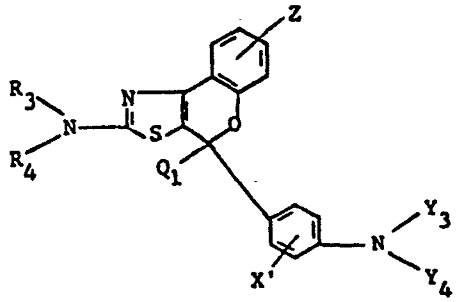

(2)

worin $R_3$, $R_4$, $Y_3$ und $Y_4$ unabhängig voneinander je Niederalkyl, Phenyl oder Benzyl und $R_3$ auch Wasserstoff oder die Substituentenpaare $R_3$ und $R_4$ und $Y_3$ und $Y_4$ unabhängig voneinander je zusammen mit dem sie verbindenden Stickstoffatom einen Pyrrolidino-, Piperidino-, Morpholino- oder Carbazolylrest, $Q_1$ Wasserstoff, Niederalkyl, Phenyl, Benzyl oder die Gruppe der Formel

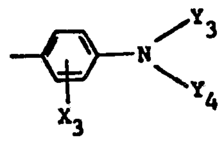

(2a)

X' und $X_3$ je Wasserstoff, Halogen, Methyl oder Niederalkoxy und Z Wasserstoff, Halogen, Niederalkyl, Niederalkoxy oder eine gegebenenfalls durch Niederalkyl, Benzyl oder Phenyl substituierte Aminogruppe bedeuten.

Von besonderem Interesse sind Benzopyranothiazole der Formel

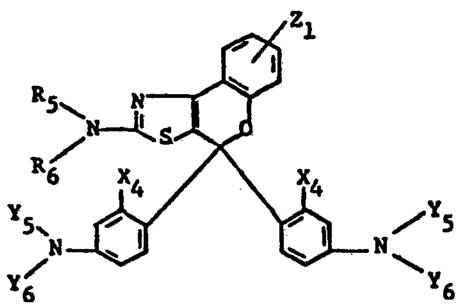

(3)

worin

$R_5$ und $R_6$ je Niederalkyl, Benzyl oder Phenyl oder zusammen mit dem sie verbindenden Stickstoffatom einen N-Carbazolylrest,

$Y_5$ und $Y_6$ je Niederalkyl oder Benzyl,

$X_4$ Wasserstoff, Methyl, Methoxy oder Aethoxy und

$Z_1$ Wasserstoff, Chlor, Methyl, Methoxy oder eine durch Niederalkyl mono- oder disubstituierte Aminogruppe bedeuten.

Unter diesen Benzopyranothiazolen der Formel (3) sind diejenigen besonders bevorzugt, bei denen $R_5$ und $R_6$ Niederalkyl oder Benzyl bedeuten.

Gut geeignete Benzopyranothiazole entsprechen vor allem der Formel

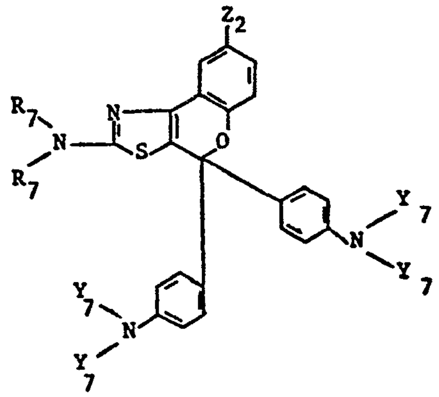

(4)

worin $R_7$ und $Y_7$ je Methyl oder Aethyl und $Z_2$ Wasserstoff oder Methyl bedeuten.

Die Benzopyranothiazole der Formeln (1) bis (4) werden dadurch hergestellt, dass man eine Verbindung der Formel

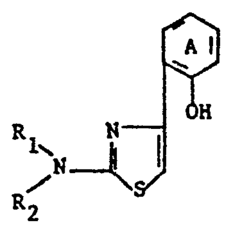

(5)

mit einer Verbindung der Formel

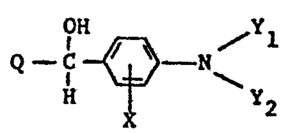

(6)

umsetzt und anschliessend oxidiert oder mit einer Ketoverbindung der Formel

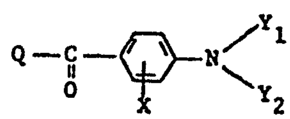

(7)

umsetzt, wobei A, $R_1$, $R_2$, Q, X, $Y_1$ und $Y_2$ die angegebene bedeutung haben.

Die Reaktion mit der Ketoverbindung der Formel (7) wird vorzugsweise so durchgeführt, dass man die Reaktionskomponenten in Anwesenheit eines sauren Entwässerungsmittels zur Reaktion bringt. Beispiele für derartige Kondensationsmittel sind Schwefelsäure, Oleum, Phosphorpentoxyd oder vorzugsweise Säurehalogenide.

Als Säurehalogenide können Säurebromide oder vorzugsweise Säurechloride der phosphorigen oder schwefligen Säure, der Phosphorsäure, der Schwefelsäure, der Kohlensäure oder Oxalsäure in Betracht kommen. Mit Vorteil verwendet man Oxalylchlorid, Oxalylbromid, Thionylchlorid, Sulfurylchlorid, Phosphortrichlorid, Phosphortribromid oder verzugsweise Phosgen oder insbesondere Phosphoroxichlorid.

Die Umsetzung der Verbindung der Formel (5) mit der Ketoverbindung der Formel (7) kann bei einer Temperatur zwischen 20 und 120°C vorgenommen werden. Es ist vorteilhaft, wasserfreie Bedingungen einzuhalten. Als Reaktionsmedium kann ein Ueberschuss des Säurehalogenids verwendet werden, jedoch kan auch ein unter den Reaktionsbedingungen inertes Lösungsmittel zugesetzt werden.

Als Lösungsmittel kommen beispielsweise in Betracht: cycloaliphatische oder aromatische Kohlenwasserstoffe, wie z.B. Cyclohexan, Benzol, Toluol oder Xylol; Chlorkohlenwasserstoffe, wie Chloroform, Tetrachlorkohlenstoff, Aethylenchlorid oder Chlorbenzole; Aether, wie Dioxan, Diäthyläther, Glykoldimethyläther oder Tetrahydrofuran.

Die Konzentration der Reaktionsteilnehmer ist nicht kritisch; man verwendet jedoch mit Vorteil je ein Moläquivalent der Reaktionskomponenten. Die Herstellung wird in der Regel so durchgeführt, dass man alle Reaktionsteilnehmer, d.h. die Verbindung der Formel (5), die Verbindung der Formel (7) und das Säurehalogenid gleichzeitig zusammengibt. Man kann jedoch auch so vorgehen, dass man zuerst die Verbindung der Formel (5) und das Säurehalogenid reagieren lässt und dann die Verbindung der

Formel (7) zugibt. Die Isolierung des Endproduktes der Formel (1) erfolgt in allgemein bekannter Weise, z.B. durch Eingiessen des Reaktionsgemisches in Eiswasser, gegebenenfalls unter Abstumpfen der Säuren mit einer alkalischen Verbindung, z.B. Alkalimetallhydroxiden oder Alkalimetallcarbonaten, Abfiltrieren des gebildeten Niederschlages, Waschen und Trocknen sowie gegebenenfalls durch Chromatographie oder Umkristallisieren des Produktes. Flüssige Endprodukte können durch Extraktion mit geeigneten organischen Lösungsmitteln gewonnen und gegebenenfalls durch Destillation gereinigt werden.

Die Kondensation der Verbindungen der Formeln (5) und (6) erfolgt zweckmässig in einem organischen Lösungsmittel, besonders in niedrigen aliphatischen Alkoholen, wie z.B. Methanol, Aethanol oder Isopropanol oder in Aethern, wie z.B. Tetrahydrofuran und vorzugsweise in Anwesenheit eines sauren Katalysators. Die Umsetzung kann schon bei Raumtemperatur (20° bis 25°C) vorgenommen werden. Zweckmässig ist jedoch die Anwendung erhöhter Temperatur, vorzugsweise 40° bis 100°C. Als saure Katalysatoren eignen sich z.B. niedere aliphatische Carbonsäuren wie Ameisensäure oder Essigsäure und anorganische Säuren wie Salzsäure, Schwefelsäure, Phosphorsäure oder Perchlorsäure. Die Reaktionsdauer hängt von der Temperatur ab und liegt in der Regel zwischen $\frac{1}{2}$ Stunde und 15 Stunden.

Die Umsetzung der Verbindungen der Formel (5) mit der Verbindung der Formel (6) ergibt unter Wasserabspaltung ein Umsetzungsprodukt der Formel

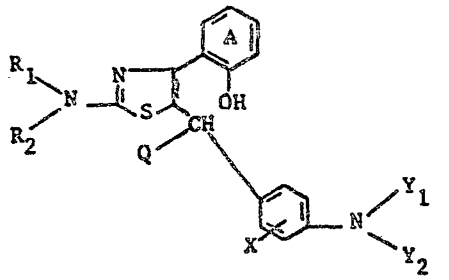

(8)

worin $R_1$, $R_2$, $Y_1$, $Y_2$, A, X und Q die angegebene Bedeutung haben.

Das erhaltene Umsetzungsprodukt der Formel (8) kann, falls erwünscht, isoliert werden.

Die Oxidation der Umsetzungsprodukte der Formel (8) zu den Benzopyranothiazolen der Formel (1) erfolgt mit Oxidationsmitteln. Geeignete Oxidationsmittel sind z.B., Chromate, Bichromate, Chlorate, Chlorite, Peroxyde, Mangandioxid, Bleidioxid, Chlor, Brom, molekularer Sauerstoff, Luft, Perborate, Permanganate, Wasserstoffperoxid und vor allem Chloranil.

Mit Vorteil arbeitet man in Gegenwart eines an der Oxidation teilnehmenden organischen Lösungsmittels. Geeignete Lösungsmittel sind wiederum niedere aliphatische Alkohole wie Aethanol, Isopropanol, Aethylenglykolmonomethyläther oder -äthyläther oder niedere aliphatische Ketone wie Aceton, Methyläthylketon oder Methylisobutylketon oder aromatische Kohlenwasserstoffe wie Benzol oder Toluol.

Die bestden Ergebnisse in Bezug auf Ausbeute und Reinheit der erhaltenen Benzopyranothiazole erzielt man mit Chloranil als bevorzugtes Oxidationsmittel, das vorzugsweise in Toluol angewandt wird.

Die Oxidationstemperatur richtet sich in der Regel nach dem Oxidationsmittel und vor allem nach dem Siedepunkt des verwendeten Lösungsmittels. Sie liegt zweckmässig zwischen 20 und 150°C, vorzugsweise 20 und 100°C. Bei Verwendung von Chloranil verläuft die Oxidation vorzugsweise bei Raumtemperatur. Die Oxidation dauert in der Regel 5 bis 30 Stunden.

Die Benzopyranothiazole der Formeln (1) bis (4) sind normalerweise farblos oder schwach gefärbt. Wenn diese Farbbildner mit einem sauren Entwickler, d.h. mit einem Elektronenakzeptor, in Kontakt gebracht werden, so ergeben sie intensive rote bis blaue Farbtöne, die ausgezeichnet lichtecht sind. Sie sind deshalb auch sehr wertvoll im Gemisch mit anderen bekannten Farbbildnern, z.B. 3,3-(Bis-aminophenyl-)phthaliden, 3,3-(Bis-Indolyl-)phthaliden, 2,6-Diaminofluoranen, Spiropyranen oder Benzoylleucomethylenblau, um blaue, marineblaue, graue oder schwarze Färbungen zu ergeben.

Die Benzopyranothiazole der Formeln (1) bis (4) zeigen sowohl auf Ton als auch auf phenolischen Unterlagen eine verbesserte Farbintensität und Lichtechtheit. Sie eignen sich vor allem als sich schnell entwickelnde Farbbildner für die Verwendung in einem druck- oder wärmeempfindlichen Aufzeichnungsmaterial, das sowohl Kopier-als auch Registriermaterial sein kann.

Ein druckempfindliches Material besteht beispielsweise aus mindestens einem Paar von Blättern, die mindestens einen Farbbildner der Formeln (1) bis (4) gelöst in einem organischen Lösungsmittel und einen festen Elektronenakzeptor als Entwickler enthalten. Der Farbbildner liefert an den Punkten, an denen er mit dem Elektronenakzeptor in Kondakt kommt, eine gefärbte Markierung.

Typische Beispiele für solche Entwickler sind Attapulgus-Ton, säureaktiviertes Bentonit (Silton-Ton), Montmorillonit, Siliciumdioxyd, Bentonit, Halloysit, Aluminiumoxid, Aluminiumsulfat, Aluminiumphosphat, Zinkchlorid, Kaolin oder irgendein beliebiger saurer Ton oder sauer reagierende, organische Verbindungen, wie z.B. gegebenenfalls ringsubstituierte Phenole, Salicylsäure oder Salicylsäureester

und deren Metallsalze, ferner ein sauer reagierendes, polymeres Material, wie z.B. ein phenolisches Polymerisat ein Alkylphenolacetylenharz, ein Maleinsäure/Kolophoniumharz oder ein teilweise oder vollständig hydrolisiertes Polymerisat von Maleinsäureanhydrid mit Styrol, Aethylen, Vinylmethyläther oder Carboxypolymethylen.

Bevorzugte Entwickler sind Attapulgus-Ton, Silton-Ton oder ein Phenolformaldehydharz.

Diese Elektronenakzeptoren werden vorzugsweise in Form einer Schicht auf die Vorderseite des Empfangsblattes aufgebracht.

Um zu verhindern, dass in dem druckempfindlichen Aufzeichnungsmaterial die Farbbildner frühzeitig aktiv werden, werden sie in der Regel von dem Elektronenakzeptor getrennt. Dies kann zweckmässig erzielt werden, indem man die Farbbildner in schaum-, schwamm- oder bienenwabenartige Strukturen einarbeitet. Vorzugsweise sind die Farbbildner in Mikrokapsein eingeschlossen, die sich in der Regel durch Druck zerbrechen lassen.

Wenn die Kapseln durch Druck, beispielsweise mittels eines Bleistiftes, zerbrochen werden und dadurch die Farbbildner-lösung auf ein benachbartes Blatt übertragen wird, das mit einerm Elektronenakzeptor beschichtet ist, wird eine farbige Stelle erzeugt. Diese Farbe ergibt sich aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Trichlordiphenyl oder eine Mischung davon mit flüssigem Paraffin, ferner Tricresylphosphat, Di-n-butylphthalat, Dioctylphthalat, Trichlorbenzol, Nitrobenzol, Trichloräthylphosphat, Kohlenwasserstofföle, wie Paraffin, alkylierte Derivate von Diphenyl, Naphthalin oder Triphenyl, Terphenyle, partiell hydriertes Terphenyl oder weitere chlorierte oder hydrierte, kondensierte, aromatische Kohlenwasserstoffe.

Die Kapselwände können durch Koazervationskräfte gleichmässig um die Tröpfchen der Farbbildnerlösung herum gebildet werden, wobei das Einkapselungsmaterial z.B. aus Gelatine und Gummiarabikum bestehen kann, wie dies z.B. in der US—PS 2 800 457 beschrieben ist. Die Kapsein können vorzugsweise auch aus einem Aminoplast oder modifizierten Aminoplasten durch Polykondensation gebildet werden, wie dies in den britischen Patentschriften 989,264, 1 156 725, 1 301 052 und 1 355 124 beschrieben wird.

Die Farbbildner der Formel (1) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf die Rückseite eines Uebertragungsblattes und der Elektronenakzeptor in Form einer Schicht auf die Vorderseite eines Empfangsblattes aufgebracht sind. Die Komponenten können aber auch in der Papierpulpe verwendet werden.

Eine andere Anordnung der Bestandteile besteht darin, dass die Farbbildner enthaltenden Mikrokapseln und der Entwickler in oder auf dem gleichen Blatt in Form einer oder mehrerer Einzelschichten oder in der Papierpulpe vorliegen.

Solche druckempfindliche Kopiermaterialien sind beispielsweise in den US-Patentschriften 2 730 457, 2 932 582, 3 418 250, 3 427 180 und 3 516 846 beschrieben. Weitere Systeme sind in den britischen Patentschriften 1 042 596, 1 042 597, 1 042 598, 1 042 599 und 1 053 935 dargestellt. Mikrokapseln welche Farbbildner der Formel (1) enthalten, eignen sich für jedes dieser Systeme sowie für andere druckempfindliche Systeme.

Die Kapseln werden vorzugsweise mittels eines geeigneten Klebstoffes auf dem Träger befestigt. Da Papier das bevorzugte Trägermaterial ist, handelt es sich bei diesen Klebstoffen hauptsächlich um Papierbeschichtungsmittel, wie Gummiarabicum, Polyvinylalkohol, Hydroxymethylcellulose, Casein, Methylcellulose oder Dextrin.

Der hier verwendete Ausdruck "Papier" umfasst nicht nur normale Papiere aus Cellulosefasern, sondern auch Papier, in denen die Cellulosefasern (teilweise oder vollständig) durch Fasern aus synthetischen Polymerisaten ersetzt sind.

Die Benzopyranothiazole der Formeln (1) bis (4) können auch als Farbbildner in einem thermoreaktiven Aufzeichnungsmaterial verwendet werden. Dieses enthält in der Regel mindestens einen Träger, einen Farbbildner, einen festen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern, oder in Messinstrumenten, verwendet. Die Bilderzeugung (Markierungserzeugung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten, sauren Tonminerale und Phenolharze, oder auch phenolische Verbindungen, wie z.B. 4-tert-Butylphenol, 4-Phenylphenol, 4-Hydroxydiphenyläther, $\alpha$-Naphthol, $\beta$-Naphthol, 4-Hydroxybenzoesäuremethylester, 4-Hydroxyacetophenon, 2,2'-Dihydroxydiphenyl, 4,4'-Isopropylidendiphenol, 4,4'-Isopropyliden-bis-(2-methylphenol), 4,4'-Bis-(hydroxyphenyl) valeriansäure, Hydrochinon, Pyrogallol, Chloroglucin, p-, m-, o-Hydroxybenzoesäure, Gallussäure, 1-Hydroxy-2-naphthoesäure sowie Borsäure und aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Benzopyranothiazole und der Entwickler in Wasser unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und eine Farbe bilden kann. Wasserlösliche oder mindestens in Wasser qeullbare Bindemittel sind z.B. hydrophile Polymerisate, wie Polyvinylalkohol, Polyacrylsäure, Hydroxyäthylcellulose, Methylcellulose, Carboxymethylcellulose, Polyacrylamid, Polyvinylpyrrolidon, Gelatine und Stärke.

Wenn der Farbbildner und der Entwickler in zwei getrennten Schichten vorliegen, können in Wasser unlösliche Bindemittel, d.h. in nicht-polaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylmethacrylate, Aethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten z.B. Talk, $TiO_2$, ZnO oder $CaCO_3$ oder auch organische Pigmente, wie z.B. Harnstoff-Formaldehydpolymerisate enthalten. Um zu bewirken, dass nur innerhalb eines begrenzten Temperaturbereiches die Farbe gebildet wird, können Substanzen, wie Harnstoff, Thioharnstoff, Acetanilid, Phthalsäureanhydrid oder andere entsprechende, schmelzbare Produkte, welche das gleichzeitige Schmelzen des Farbbildners und des Entwicklers induzieren, zugesetzt werden.

In den folgenden Beispielen beziehen sich die angegebenen Prozentsätze, wenn nichts anderes angegeben ist, auf das Gewicht.

BEISPIEL 1

17,5 g 2-Chloroacetyl-4-methyl-phenol und 10,9 g N,N,-Di-methylthioharnstoff werden 20 Minuten auf 110°C erhitzt. Nach dem Abkühlen wird die erstarrte Masse aus wässrigem Methanol umkristallisiert. Es werden 14,2 g (96% der Theorie) 2-Dimethylamino-4-(2'-hydroxy-5'-methylphenyl)-thiazol der Formel (101) erhalten. Schmelzpunkt 108—110°C.

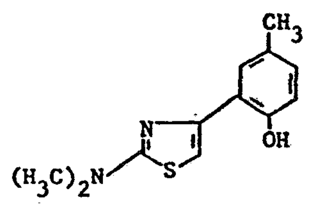

(101)

Eine Mischung von 5,4 g 4,4'-Bis-(dimethylamino)-benzhydrol ("Michler's Hydrol"), 4,9 g der Verbindung der Formel (101), 60 ml Aethanol und 1 ml Eisessig werden 18 Stunden am Rückfluss erhitzt. Man kühlt das Gemisch auf Raumtemperatur ab, filtriert den Niederschlag ab und wäscht ihn mit wenig Methanol. Man erhält 6,1 g (63% der Theorie)2 - Dimethylamino - 4 - (2' - hydroxy - 5' - methylphenyl) - 5 - [bis - (4'' - dimethylaminophenyl) - methyl] - thiazol der Formel (102). Schmelzpunkt 189—191°C.

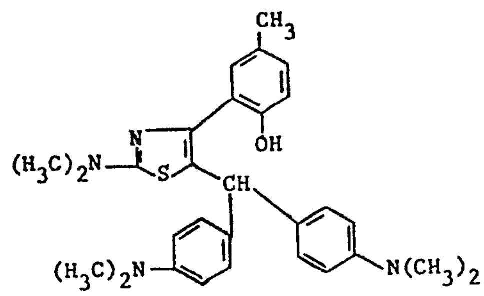

(102)

Eine Mischung von 4,86 g der Verbindung der Formel (102), 2,52 g Chloranil und 60 ml Toluol wird 20 Stunden bei Raumtemperatur gerührt. Der entstandene Niederschlag wird abfiltriert und mit 10 ml Toluol gewaschen. Man erhält 3,1 g (64% der Theorie) einer Benzopyranoverbindung der Formel (103), Schmelzpunkt 227—229°C. Auf Silton-Ton entwickelt dieser Farbbildner eine blaue Farbe von $\lambda$ max. 648 nm.

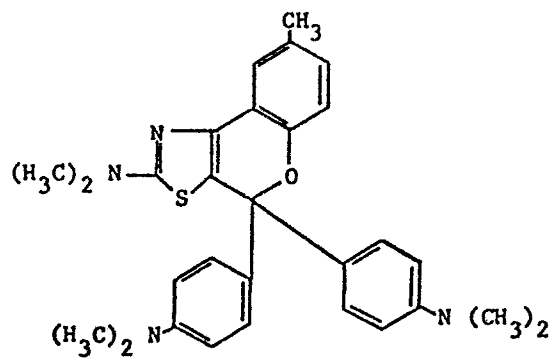

(103)

Auf gleiche Weise wie in Beispiel 1 beschrieben, erhält man die in der folgenden Tabelle aufgeführten Farbbildner der Formel

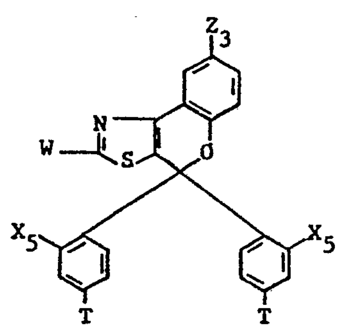

(104)

TABELLE

| Beispiel Nr. | W | $Z_3$ | T | $X_5$ | Farbe auf Silton-Ton |
|---|---|---|---|---|---|
| 2 | $-N(C_2H_5)_2$ | H | $-N(CH_3)_2$ | H | blau |
| 3 | $-N(C_2H_5)_2$ | $CH_3$ | $-N(CH_3)_2$ | H | blau |
| 4 | $-N(C_2H_5)_2$ | $CH_3$ | $-N(CH_3)_2$ | H | blau |
| 5 | $-N(CH_3)_2$ | Cl | $-N(C_2H_5)_2$ | H | blau |
| 6 | $-N(CH_2$ 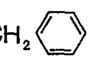 $)_2$ | $CH_3$ | $-N(C_2H_5)_2$ | H | blau |
| 7 | $-N(CH_3)_2$ | $-OCH_3$ | $-N(CH_3)_2$ | H | blau |
| 8 | 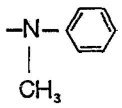 | $CH_3$ | $-N(CH_3)_2$ | H | blau |
| 9 | $-N(CH_3)_2$ | $CH_3$ | $-N(CH_2$ 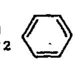 $)_2$ | H | blau |
| 10 | $-N(CH_3)_2$ | $CH_3$ | $-N(C_2H_5)_2$ | $-OCH_3$ | blau |
| 11 | $-N(CH_3)_2$ | $-N(CH_3)_2$ | $-N(C_2H_5)_2$ | H | blau |
| 12 | 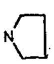 | $CH_3$ | $-N(C_2H_5)_2$ | H | blau |
| 13 | $-N(CH_3)_2$ | $CH_3$ | 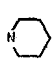 | H | blau |
| 14 | $-N(CH_3)_2$ | $CH_3$ | $-N(C_2H_5)_2$ | $-OC_2H_5$ | blau |

BEISPIEL 15

*Herstellung eines druckempfindlichen Kopierpapiers*

Eine Lösung von 3 g des Benzopyranothiazols der Formel (103) in 97 g partiell hydriertem Terphenyl wird in einer Lösung von 12 g Schweinehautgelatine in 88 g Wasser von 50°C emulgiert. Sodann wird eine Lösung von 12 g Gummiarabicum in 88 g Wasser von 50°C zugegeben und hierauf 200 ml Wasser von 50°C zugefügt. Die erhaltene Emulsion wird in 600 g Eiswasser eingegossen und gekühlt, wobei die Koazervation bewirkt wird. Mit der dabei erhaltenen Suspension der Mikrokapseln wird ein Blatt Papier beschichtet und getrocknet, Ein zweites Blatt Papier wird mit Silton-Ton beschichtet. Das erste Blatt und das mit Silton-Ton beschichtete Papier werden mit den Beschichtungen benachbart aufeinander gelegt.

Durch Schreiben mit der Hand oder mit der Schreibmaschine auf dem ersten Blatt wird Druck ausgeübt und es entwickelt sich auf dem mit Ton beschichteten Blatt eine intensive blaue Kopie, die ausgezeichnet lichtecht ist. Entsprechende intensive blaue Kopien werden auch bei Verwendung jedes der anderen in den Beispielen 2 bis 14 angegebenen Farbbildnern.

BEISPIEL 16

*Herstellung eines thermoreaktiven Papiers*

6 g einer wässrigen Dispersion, die 1,57% des Benzopyranothiazols der Formel (103) und 6,7% Polyvinylalkohol enthält, werden mit 134 g einer wässrigen Dispersion gemischt, die 14% 4,4-Isopropylidendiphenol, 8% Attapulgus-Ton und 6% Polyvinylalkohol enthält. Dieses Gemisch wird auf ein Papier aufgetragen und getrocknet. Durch Berührung des Papiers mit einem erhitzten Kugelschreiber

wird eine intensive blaue Farbe erhalten, die eine ausgezeichnete Lichtechtheit hat.

Intensive blaue Farben können auch bei Verwendung jedes der anderen Farbbildner der Beispiele 2 bis 14 erhalten werden.

**Patentansprüche**

1. Benzopyranothiazole, dadurch gekennzeichnet, dass diese der Formel

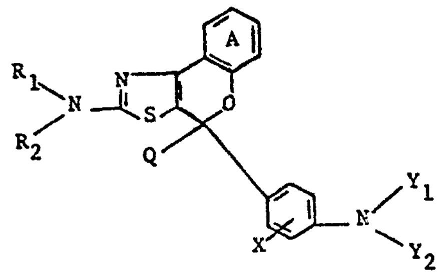

(1)

entsprechen, worin

$R_1$ und $R_2$ unabhängig voneinander je Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl, Benzyl oder durch Halogen, Niederalkyl oder Niederalkoxy substituiertes Phenyl oder Benzyl oder

$R_1$ und $R_2$ zusammen mit dem sie verbindenden Stickstoffatom einen 5- oder 6-gliedrigen, heterocyclischen Rest oder einen N-Carbazolylrest,

Q Wasserstoff, Niederalkyl, Benzyl oder die Gruppen der Formeln (1a) oder (1b)

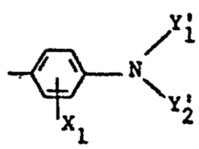

oder

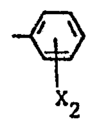

(1a) (1b)

X, $X_1$ und $X_2$ je Wasserstoff, Halogen, Niederalkyl oder Niederalkoxy, $Y_1$, $Y_2$, $Y_1'$ und $Y_2'$ je Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl, Benzyl oder durch Halogen, Niederalkyl oder Niederalkoxy substituiertes Phenyl oder Benzyl oder die Substituentenpaare $Y_1$ und $Y_2$ und $Y_1'$ und $Y_2'$ unabhängig voneinander zusammen mit dem sie verbindenden Stickstoffatom einen 5- oder 6-gliedrigen, heterocyclischen Rest oder einen N-Carbazolylrest bedeuten und der Ring A unsubstituiert oder durch Halogen, Nitro, Niederalkyl, Niederalkoxy, Phenoxy oder eine gegebenenfalls durch Niederalkyl, Phenyl oder Benzyl substituierte Aminogruppe substituiert ist.

2. Benzopyranothiazole gemäss Anspruch 1, dadurch gekennzeichnet, dass diese der Formel

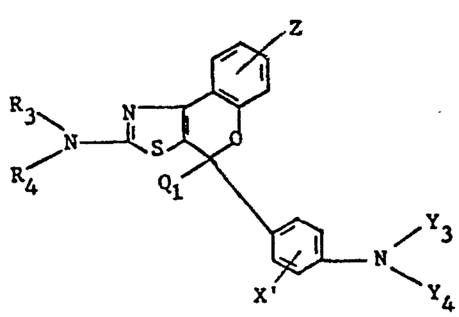

(2)

entsprechen, worin $R_3$, $R_4$, $Y_3$ und $Y_4$ unabhängig voneinander je Niederalkyl, Phenyl oder Benzyl und $R_3$ auch Wasserstoff oder die Substituentenpaare $R_3$ und $R_4$ und $Y_3$ und $Y_4$ je zusammen mit dem sie verbinenden Stickstoffatom einen Pyrrolidino-, Piperidino-, Morpholino- oder Carbazolyl-rest, $Q_1$ Wasserstoff, Niederalkyl, Phenyl, Benzyl oder die Gruppe der Formel

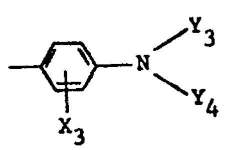

(2a)

X' und $X_3$ je Wasserstoff, Halogen, Methyl oder Niederalkoxy und Z Wasserstoff, Halogen, Niederalkyl, Niederalkoxy oder eine gegebenenfalls durch Niederalkyl, Benzyl oder Phenyl substituierte Aminogruppe bedeuten.

3. Benzopyranothiazole gemäss Anspruch 2, dadurch gekennzeichnet, dass diese der Formel

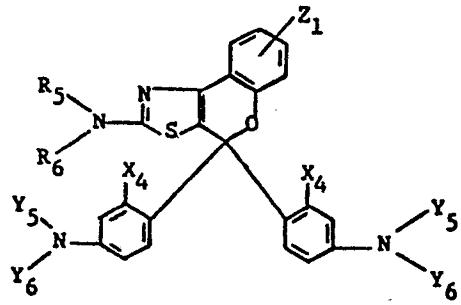

(3)

entsprechen, worin $R_5$ und $R_6$ je Niederalkyl, Benzyl oder Phenyl oder $R_5$ und $R_6$ zusammen mit dem sie verbindenden Stickstoffatom einen N-Carbazolylrest, $Y_5$ und $Y_6$ je Niederalkyl oder Benzyl, $X_4$ Wasserstoff, Methyl, Methoxy oder Aethoxy und $Z_1$ Wasserstoff, Chlor, Methyl, Methoxy oder eine durch Niederalkyl mono- oder disubstituierte Aminogruppe bedeuten.

4. Benzopyranothiazole gemäss Anspruch 3, dadurch gekennzeichnet, dass diese der Formel (3) entsprechen, worin $R_5$ und $R_6$ Niederalkyl oder Benzyl bedeuten.

5. Benzopyranothiazole gemäss Anspruch 3, dadurch gekennzeichnet, dass diese der Formel

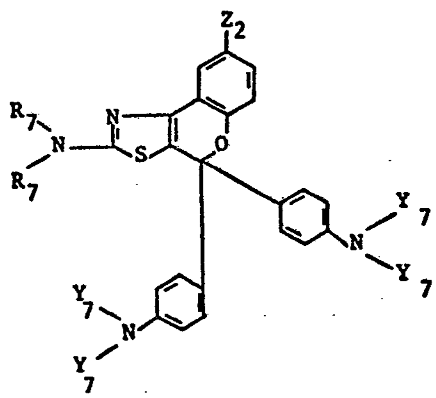

(4)

entsprechen, worin $R_7$ und $Y_7$ je Methyl oder Aethyl und $Z_2$ Wasserstoff oder Methyl bedeuten.

6. Verfahren zur Herstellung von Benzopyranothiazolen der im Anspruch 1 angegebenen Formel, dadurch gekennzeichnet, dass man eine Verbindung der Formel

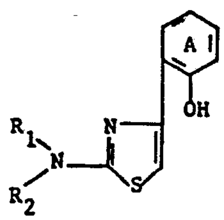

(5)

mit einer Verbindung der Formel

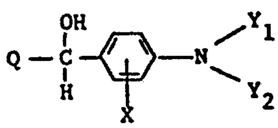

(6)

umsetzt und anschliessend oxidiert oder mit einer Ketoverbindung der Formel

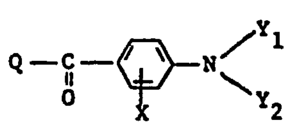

(7)

umsetzt, wobei A, $R_1$, $R_2$, Q, X, $Y_1$ und $Y_2$, die im Anspruch 1 angegebene Bedeutung haben.

7. Verwendung eines benzopyranothiazols der in einem der Ansprüche 1 bis 5 angegebenen Formel als Farbbildner in einem druckempfindlichen oder wärmeempfindlichen Aufzeichnungsmaterial.

8. Druck- oder wärmeempfindliches Aufzeichnungsmaterial, dadurch gekennzeichnet, dass es in seinem Farbreaktantensystem als Farbbildner mindestens ein Benzopyranothiazol der in einem der Ansprüche 1 bis 5 angegebenen Formel enthält.

9. Druckempfindliches Aufzeichnungsmaterial nach Anspruch 8, dadurch gekennzeichnet, dass es mindestens ein Benzopyranothiazol der in einem der Ansprüche 1 bis 5 angegebenen Formel als Farbbildner, gelöst in einem organischen Lösungsmittel, und einen festen Elektronenakzeptor enthält.

## Claims

1. A benzopyranothiazole characterised by the formula

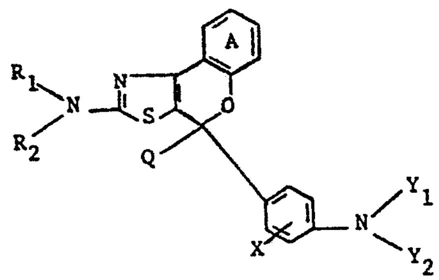

(1)

wherein each of $R_1$ and $R_2$ independently represents hydrogen, alkyl containing at most 12 carbon atoms which is unsubstituted or substituted by halogen, hydroxyl, cyano or lower alkoxy, or represents cycloalkyl, phenyl, benzyl, or phenyl or benzyl which is substituted by halogen, lower alkyl or lower alkoxy, or $R_1$ and $R_2$ together with the nitrogen atom to which they are attached represent a 5- or 6-membered heterocyclic radical or a N-carbazolyl radical, Q represents hydrogen, lower alkyl, benzyl or the groups of the formulae (1a) or (1b)

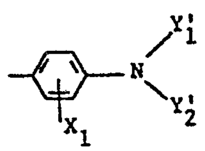

or

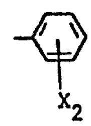

(1a) (1b)

each of X, $X_1$ and $X_2$ represents hydrogen, halogen, lower alkyl or lower alkoxy, each of $Y_1$, $Y_2$, $Y_1'$ and $Y_2'$ represents hydrogen, alkyl containing at most 12 carbon atoms which is unsubstituted or substituted by halogen, hydroxyl, cyano or lower alkoxy, or represents cycloalkyl, phenyl, benzyl, or phenyl or benzyl which is substituted by halogen, lower alkyl or lower alkoxy, or each of the pair of substituents $Y_1$ and $Y_2$ and $Y_1'$ and $Y_2'$, together with the nitrogen atom to said pair is attached, independently represents a 5- or 6- membered heterocyclic radical or a N-carbazolyl radical and the ring A is unsubstituted or substituted by halogen, nitro, lower alkyl, lower alkoxy, phenoxy or an amino group which is unsubstituted or substituted by lower alkyl, phenyl or benzyl.

2. A benzopyranothiazole according to claim 1 characterised by the formula

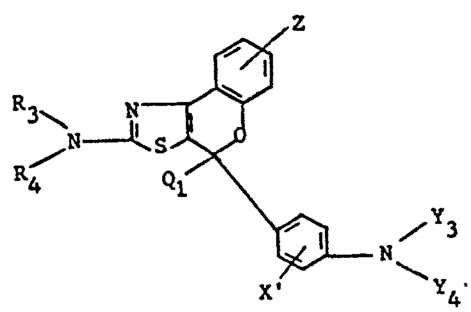

(2)

wherein $R_3$, $R_4$, $Y_3$ and $Y_4$, each independently of the other, represent lower alkyl, phenyl or benzyl and

$R_3$ also represents hydrogen, or each of the pair of substituents $R_3$ and $R_4$ and $Y_3$ and $Y_4$, together with the nitrogen atom to which said pair is attached, independently represents a pyrrolidino, piperidino, morpholino or carbazolyl radical, $Q_1$ represents hydrogen, lower alkyl, phenyl, benzyl or the group of the formula

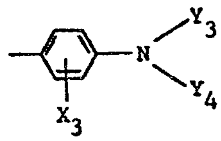

(2a)

each of X' and $X_3$ represents hydrogen, halogen, methyl or lower alkoxy and Z represents hydrogen, halogen, lower alkyl, lower alkoxy or an amino group which is unsubstituted or substituted by lower alkyl, benzyl or phenyl.

3. A benzopyranothiazole according to claim 2 characterised by the formula

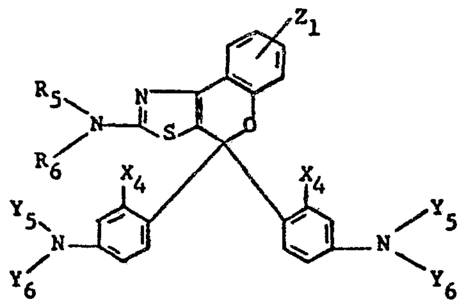

(3)

wherein each of $R_5$ and $R_6$ represents lower alkyl, benzyl or phenyl, or together with the nitrogen atom to which they are attached represent a N-carbazolyl radical, each of $Y_5$ and $Y_6$ represents lower alkyl or benzyl, $X_4$ represents hydrogen, methyl, methoxy or ethoxy, and $Z_1$ represents hydrogen, chlorine, methyl, methoxy or an amino group which is mono- or disubstituted by lower alkyl.

4. A benzopyranothiazole according to claim 3 characterised by the formula (3), wherein $R_5$ and $R_6$ represent lower alkyl or benzyl.

5. A benzopyranothiazole according to claim 3 characterised by the formula

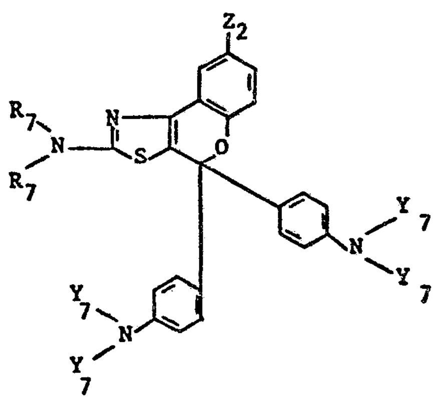

(4)

wherein each of $R_7$ and $Y_7$ represents methyl or ethyl and $Z_2$ represents hydrogen or methyl.

6. A process for the production of a benzopyranothiazole of the formula indicated in claim 1, characterised in that a compound of the formula

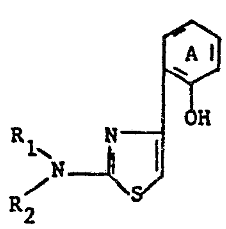

(5)

wherein A, $R_1$ and $R_2$ are as defined in claim 1, is reacted with a compound of the formula

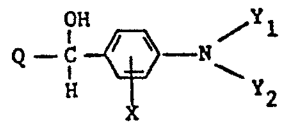

(6)

wherein Q, X, $Y_1$ and $Y_2$ are as defined in claim 1, and the reaction product is subsequently oxidised, or the compound of the formula (5) is reacted with a keto compound of the formula

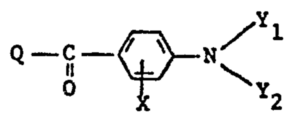

(7)

wherein Q, X, $Y_1$ and $Y_2$ are as defined in claim 1.

7. Use of a benzopyranothiazole of the formula indicated in any one of claims 1 to 5 as colour former in a pressure-sensitive or heat-sensitive recording material.

8. A pressure-sensitive or heat-sensitive recording material, characterised in that it contains as colour former in its colour reactant system at least one benzopyranothiazole of the formula indicated in any one of claims 1 to 5.

9. A pressure-sensitive recording material according to claim 8, characterised in that it contains as colour former at least one benzopyranothiazole of the formula indicated in any one of claims 1 to 5, dissolved in an organic solvent, and at least one solid electron acceptor.

**Revendications**

1. Benzopyranothiazoles, caractérisés par le fait qu'ils correspondent à la formule:

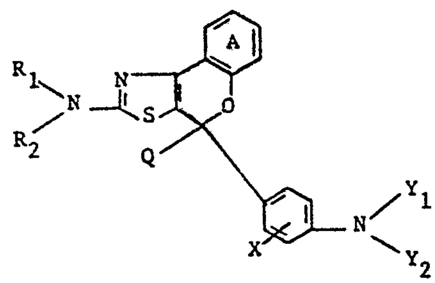

(1)

dans laquelle $R_1$ et $R_2$ indépendamment l'un de l'autre représentent chacun un atome d'hydrogène, un groupe alkyle ayant au plus 12 atomes de carbone, non substitué ou substitué par de l'halogène, des radicaux hydroxyle, cyano ou alcoxy inférieur, un groupe cycloalkyle, phényle, benzyle, ou un groupe phényle ou benzyle substitués par de l'halogène, des radicaux alkyle inférieur ou alcoxy inférieur, ou bien

$R_1$ et $R_2$ conjointement à l'atome d'azote auquel ils sont reliés, représentent un reste hétérocyclique à 5 ou 6 chaînons ou bien un reste N-carbazolyle;

Q est un atome d'hydrogène, un groupe alkyle inférieur, benzyle ou bien les groupes des formules (1a) ou (1b)

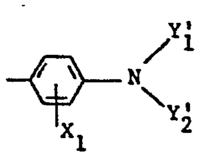

ou

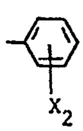

(1a) (1b)

X, $X_1$ et $X_2$ désignent chacun un atome d'hydrogène, un atome d'halogène, un groupe alkyle inférieur ou alcoxy inférieur; $Y_1$, $Y_2$, $Y'_1$ et $Y'_2$ désignent chacun un atome d'hydrogène, un groupe alkyle ayant au plus 12 atomes de carbone, non substitué ou substitué par de l'halogène, des radicaux hydroxyle, cyano ou alcoxy inférieur, un groupe cycloalkyle, phényle, benzyle, ou bien un groupe phényle ou benzyle substitué par de l'halogène, des radicaux alkyle inférieur ou alcoxy inférieur, ou bien les paires de substituants $Y_1$ et $Y_2$, et $Y'_1$ et $Y'_2$, indépendamment l'une de l'autre, forment avec l'atome d'azote auquel chacune est reliée, un reste hétérocyclique à 5 ou 6 chaînons ou un reste N-carbazolyle, et le noyau A est non substitué ou bien est substitué par de l'halogène, des radicaux nitro, alkyle inférieur, phénoxy ou par un radical amino substitué éventuellement par des restes alkyle inférieur, phényle ou benzyle.

2. Benzopyranothiazoles selon la revendication 1, caractérisés par le fait qu'ils correspondent à la formule:

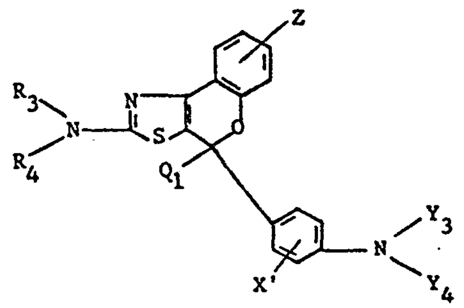

(2)

dans laquelle $R_3$, $R_4$, $Y_3$ et $Y_4$ indépendamment les uns des autres représentent chacun un groupe alkyle inférieur, phényle ou benzyle, et $R_3$ représente aussi un atome d'hydrogène, ou bien les paires de substituants $R_3$ et $R_4$, et $Y_3$ et $Y_4$ forment chacune avec l'atome d'azote auquel elle est reliée, un reste pyrrolidino, pipéridino, morpholino ou carbazolyle; $Q_1$ désigne un atome d'hydrogène, un groupe alkyle inférieur, phényle, benzyle, ou le groupe de formule:

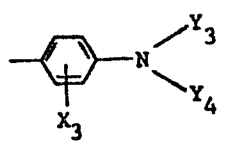

X' et $X_3$ désignent chacun un atome d'hydrogène, un atome d'halogène, un groupe méthyle ou alcoxy inférieur, et Z représente un atome d'hydrogène, un atome d'halogène, un groupe alkyle inférieur, alcoxy inférieur ou un groupe amino éventuellement substitué par les radicaux alkyle inférieur, benzyle ou phenyle.

3. Benzopyranothiazoles selon la revendication 2, caractérisés par le fait qu'ils correspondent à la formule

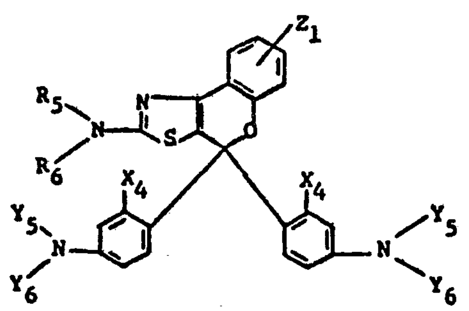

(3)

dans laquelle $R_5$ et $R_6$ désignent chacun un groupe alkyle inférieur, benzyle ou phényle, ou bien $R_5$ et $R_6$ forment avec l'atome d'azote auquel ils sont reliés, un reste N-carbazolyle; $Y_5$ et $Y_6$ représentent chacun un groupe alkyle inférieur ou benzyle; $X_4$ désigne un atome d'hydrogène, un groupe méthyle, méthoxy ou éthoxy, et $Z_1$ représente un atome d'hydrogène, de chlore, un groupe méthyle, méthoxy ou un groupe amino mono-substitué ou di-substitué par des radicaux alkyle inférieur.

4. Benzopyranothiazoles selon la revendication 3, caractérisés par le fait qu'ils correspondent à la formule (3) dans laquelle $R_5$ et $R_6$ désignent des groupes alkyle inférieur ou benzyle.

5. Benzopyranothiazoles selon la revendication 3, caractérisés par le fait qu'ils correspondent à la formule

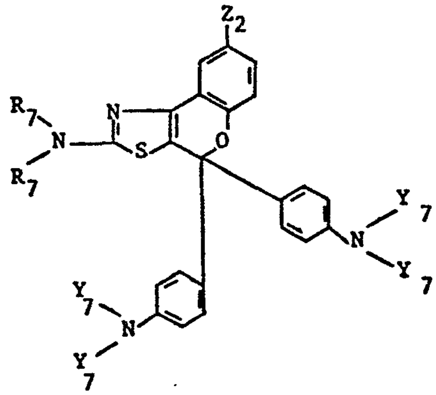

(4)

et $R_7$ et $Y_7$ désignent chacun un groupe méthyle ou éthyle, et $Z_2$ désigne un atome d'hydrogène ou un groupe méthyle.

6. procédé pour la préparation de benzopyranothiazoles ayant la formule donnée dans la revendication 1, caractérisé par la fait qu'on fait réagir un composé de formule:

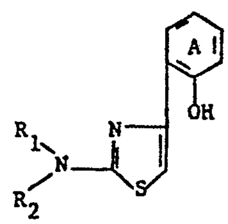

(5)

avec un composé de formule

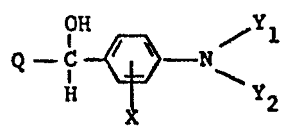

(6)

puis qu'on oxyde le produit obtenu ou bien qu'on fait réagir un composé de formule (5) avec un composé cétonique de formule:

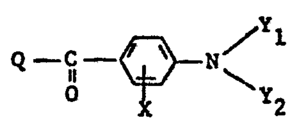

(7)

A, $R_1$, $R_2$, Q, X, $Y_1$ et $Y_2$, ayant les significations données dans la revendication 1.

7. Utilisation d'un benzopyranothiazole ayant les formules mentionnées dans l'une quelconque des revendications 1 à 5, comme chromogène dans une matière pour enregistrement sensible à la pression ou thermosensible.

8. Matière pour enregistrement sensible à la pression ou thermosensible, caractérisée par le fait qu'elle contient dans son système réactif chromogène, comme chromogène, au moins un benzopyranothiazole ayant la formule donnée dans l'une quelconque des revendications 1 à 5.

9. Matière pour enregistrement sensible à la pression selon la revendication 8, caractérisée par le fait qu'elle contient au moins un benzopyranothiazole ayant la formule donnée dans l'une quelconque des revendications 1 à 5, comme chromogène, dissous dans un solvant organique, et un accepteur d'électrons solide.